# EUROPEAN PATENT SPECIFICATION

(11) **EP 2 020 243 B1**
(45) Date of publication and mention of the grant of the patent: **15.08.2018**
(21) Application number: 08166780.0
(22) Date of filing: 27.05.2003
(51) Int. Cl.: A61K 47/10, A61K 47/14, A61K 47/06, A61K 47/32, A61K 47/44, A61K 31/44

(54) **Topically applicable pharmaceutical preparation**
Topisch anwendbare pharmazeutische Zubereitung
Préparation pharmaceutique applicable de manière topique

(30) Priority: 28.05.2002 DE 10223828; 14.03.2003 DE 10311613; 28.05.2002 EP 02011830
(43) Date of publication of application: 04.02.2009
(62) Divisional of application: 03755048.0
(73) Proprietor: AstraZeneca AB, 151 85 Södertälje (SE)
(72) Inventor: Linder, Rudolf, 78464 Konstanz (DE); Bolle, Christina, 4053 Basel (CH)
(74) Representative: AstraZeneca

(56) References cited:
- EP-A1- 0 719 561
- WO-A-00/18388
- WO-A-01/32165
- WO-A-02/38155
- WO-A-95/01338
- WO-A1-03/039552
- US-A- 4 753 945
- US-A- 5 011 843
- US-A- 5 914 334
- US-B1- 6 174 878
- REID P: "ROFLUMILAST" CURRENT OPINION IN INVESTIGATIONAL DRUGS, CURRENT DRUGS, LONDON, GB, vol. 3, no. 8, August 2002 (2002-08), pages 1165-1170, XP001119630 ISSN: 0967-8298
- Bundschuh: "In vivo efficacy in airway disease models of roflumilast, a novel orally active PDE4 inhibitor", Journal of Pharmacology and Experimental Therapeutics, vol. 297, no. 1, 1 January 2001 (2001-01-01), page 280, XP55004687, ISSN: 0022-3565

## Description

### Technical field

The present invention relates to the field of pharmaceutical technology and describes a topically applicable pharmaceutical preparation consisting of as active ingredient a compound selected from the group consisting of roflumilast, salts of roflumilast, the N-oxide of the pyridine residue of roflumilast or salts thereof, together with one or more pharmaceutical carriers and/or excipients suitable for topical administration. The invention additionally relates to the use of the topically applicable pharmaceutical preparation for the systemic treatment of diseases regarded as treatable or preventable through use of PDE 4 inhibitors.

### Prior art

Cyclic nucleotide phosphodiesterase (PDE) inhibitors (specifically of type 4) are currently of special interest as a new generation of active ingredients for treating inflammatory disorders, especially disorders of the airways such as asthma or airway obstructions (such as, for example, COPD = chronic obstructive pulmonary disease). A number of PDE 4 inhibitors are currently undergoing advanced clinical testing, including a dosage form for oral administration comprising the active ingredient N-(3,5-di-chloropyrid-4-yl)-3-cyclopropylmethoxy-4-difluoromethoxybenzamide (INN: roflumilast). This and other compounds with a benzamide structure and their use as cyclic nucleotide phosphodiesterase (PDE) inhibitors are described in WO 95/01338. These active ingredients are proposed in WO 95/01338 also for the treatment of certain disorders of the skin (such as, for example, dermatoses). WO 00/53182 proposes the use of roflumilast or its N-oxide for the treatment of multiple sclerosis.

For treating disorders of the skin it is desirable to provide the active pharmaceutical ingredient in a pharmaceutical preparation suitable for topical application. As the skilled person is aware, however, the provision of dosage forms for topical application may prove to be extremely difficult or is impossible if the intention is to administer an active ingredient which has a very low solubility. Thus, for example, the solubility in water found for the PDE 4 inhibitor N-(3,5-dichloropyrid-4-yl)-3-cyclopropylmethoxy-4-difluoromethoxybenzamide (INN: roflumilast), which is described in WO 95/01338, is only 0.53 mg/l at 21°C.

### Description of the invention

It has now been found, surprisingly, that topically applicable pharmaceutical preparations comprising the slightly soluble PDE 4 inhibitor roflumilast show a very good effect in the treatment of dermatoses on local, dermal application. Also found, entirely surprisingly, besides the local effect, is an excellent systemic effect which is comparable with that of an oral dosage form.

A first aspect of the invention is therefore a topically applicable pharmaceutical preparation consisting of an active pharmaceutical ingredient together with one or more pharmaceutical carriers and/or excipients suitable for topical administration, wherein:
i) the active pharmaceutical ingredient is a compound selected from the group consisting of roflumilast, salts of roflumilast, the N-oxide of roflumilast and salts thereof,
ii) the proportion of active pharmaceutical ingredient is up to 1 % by weight of the finished pharmaceutical preparation, and
iii) the topically applicable pharmaceutical preparation is a semisolid dosage form selected from the group of ointments (e.g. solution ointment, suspension ointment), creams, gels or pastes.

Roflumilast is the INN for a compound of the formula I 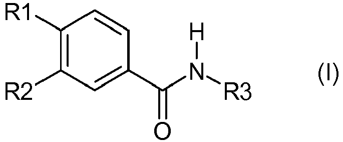 in which
- R1: is difluoromethoxy,
- R2: is cyclopropylmethoxy and
- R3: is 3,5-dichloropyrid-4-yl.

This compound has the chemical name N-(3,5-dichloropyrid-4-yl)-3-cyclopropylmethoxy-4-difluoro-methoxybenzamide (INN: roflumilast). The N-oxide of roflumilast has the chemical name 3-cyclopropylmethoxy-4-difluoromethoxy-N-(3,5-dichloropyrid-4-yl 1-oxide)benzamide.

This compound of the formula I, its salts, the N-oxide, its salts and the use of these compounds as phosphodiesterase (PDE) 4 inhibitors are described in the international patent application WO 95/01338.

Salts suitable for compounds of the formula I - depending on the substitution - are all acid addition salts but, in particular, all salts with bases. Particular mention may be made of the pharmacologically acceptable salts of the inorganic and organic acids and bases normally used in pharmaceutical technology. Pharmacologically unacceptable salts which, for example, may be the initial products of the process for preparing the compounds of the invention on the industrial scale are converted into pharmacologically acceptable salts by processes known to the skilled worker. Those suitable on the one hand are water-soluble and water-insoluble acid addition salts with acids such as, for example, hydrochloric acid, hydrobromic acid, phosphoric acid, nitric acid, sulfuric acid, acetic acid, citric acid, D-gluconic acid, benzoic acid, 2-(4-hydroxybenzoyl)benzoic acid, butyric acid, sulphosalicylic acid, maleic acid, lauric acid, malic acid, fumaric acid, succinic acid, oxalic acid, tartaric acid, embonic acid, stearic acid, toluenesulphonic acid, methanesulphonic acid, or 3-hydroxy-2-naphthoic acid, the acids being employed to prepare the salts in the equimolar ratio of amounts, or one differing therefrom - depending on whether the acid is monobasic or polybasic and depending on which salt is desired.

On the other hand, salts with bases are also particularly suitable. Examples of basic salts which may be mentioned are lithium, sodium, potassium, calcium, aluminium, magnesium, titanium, ammonium, meglumine or guanidinium salts, once again the bases being employed to prepare the salts in the equimolar ratio of amounts or one differing therefrom.

The proportion (in per cent by weight based on the weight of the finished pharmaceutical preparation; w/w) of active pharmaceutical ingredient in the pharmaceutical preparation of the invention is up to 1 % by weight.

The pharmaceutical carriers and/or excipients suitable for topical administration are preferably according to the invention conventional carriers and/or excipients known to the skilled person in connection with pharmaceutical preparations for dermal administration (= dermatologicals). Examples which may be mentioned are carriers and/or excipients which are suitable for producing dusting powders, emulsions, suspensions, sprays, oils, ointments, greasy ointments, creams, pastes, gels, foams or solutions, and transdermal therapeutic systems.

The topical pharmaceutical preparation of the invention can be produced by processes familiar to the skilled person.

Conventional dermatologicals and their production, and preferred carriers and/or excipients for the individual pharmaceutical preparations are described, for example, in the textbook "Pharmazeutische Technologie" (Sucker, Fuchs, Speiser, Georg Thieme Verlag, 1978 from page 629).

The topical pharmaceutical preparation of the invention is a semisolid dosage form selected from the group of ointments (e.g. solution ointment, suspension ointment), creams, gels or pastes.

Oil-in-water or water-in-oil emulsions are normally referred to as creams. Chiefly used for the oily phase are fatty alcohols, e.g. lauryl, cetyl or stearyl alcohol, fatty acids, e.g. palmitic or stearic acid, liquid or solid paraffins or ozokerite, liquid to solid waxes, e.g. isopropyl myristate, natural or partially synthetic fat, e.g. coconut fatty acid triglyceride, hardened oils, e.g. hydrogenated peanut or castor oil, or fatty acid partial esters of glycerol, e.g. glycerol monostearate or glycerol distearate. Suitable emulsifiers are surface-active substances, e.g. nonionic surfactants, e.g. fatty acid esters of polyalcohols or ethylene oxide adducts thereof, such as polyglycerol fatty acid esters or polyoxyethylene sorbitan fatty acid esters (Tween®: ICI,) sorbitan fatty acid esters (Span®: ICI), such as, for example, sorbitan oleate and/or sorbitan isostearate, sterols, also polyoxyethylene fatty alcohol ethers or fatty acid esters, or anionic surfactants such as alkali metal salts of fatty alcohol sulphates, e.g. sodium lauryl sulphate, sodium cetyl sulphate or sodium stearyl sulphate, which are normally used in the presence of said fatty alcohols, e.g. cetyl alcohol or stearyl alcohol. It is possible to add to the aqueous phase inter alia agents which prevent the cream drying out, e.g. polyalcohols such as glycerol, sorbitol, propylene glycol and/or polyethylene glycols, also preservatives, fragrances etc.

Ointments may be anhydrous and contain as base the paraffins which are suitable for topical use and are liquid at body temperature, especially low-viscosity paraffin, also the said natural or partially synthetic fats, e.g. coconut fatty acid triglyceride, hardened oils, e.g. hydrogenated peanut or castor oil, fatty acid partial esters of glycerol, e.g. glycerol monostearate and distearate, silicones, e.g. polydimethylsiloxanes, e.g. hexamethyldisiloxane or octamethyltrisiloxane, and, for example, the fatty alcohols mentioned in connection with the hydrous creams and increasing the water uptake capacity, and sterols, wool waxes, other emulsifiers and/or other additives.

In the case of gels, a distinction is made between hydrous, anhydrous and low water-content gels, which consist of swellable, gel-forming material. Chiefly suitable are transparent hydrogels based on inorganic or organic macromolecules. Macromolecular inorganic components with gel-forming properties are predominantly hydrous or water-absorbing silicates such as aluminium silicates, e.g. bentonite, magnesium-aluminium silicates, e.g. Veegum® - Vanderbilt Exp. Corp., or colloidal silica, e.g. Aerosil® - Degussa. Examples of macromolecular organic substances used are natural, semisynthetic or synthetic polymers. Natural and semisynthetic polymers are derived, for example, from polysaccharides with different carbohydrate units, such as cellulose, starch, tragacanth, gum arabic, agar-agar, gelatin, alginic acid and salts thereof, e.g. sodium alginate and derivatives thereof, lower alkylcellulose, for example methyl- or ethylcellulose, carboxy- or hydroxy-lower-alkylcellulose, e.g. carboxymethyl- or hydroxypropylcellulose. The units of synthetic, gel-forming polymers are, for example, unsaturated, substituted aliphatic compounds such as vinyl alcohol, vinylpyrrolidone, acrylic or methacrylic acid. Examples to be mentioned of such polymers are polyvinyl alcohol derivatives such as Polyviol®-Wacker, polyvinylpyrrolidones, such as Kollidon® - BASF or Polyplasdon® - General Aniline, polyacrylates and polymethacrylates, such as Rohagit S® - Rohm und Haas. It is possible to add conventional additives such as preservatives or fragrances to the gels.

Pastes are creams or ointments with the constituents mentioned hereinbefore and secretion-absorbing dusting powder constituents such as metal oxides, e.g. titanium oxide or zinc oxide, also talc and/or aluminium silicates, which have the task of binding moisture or secretions.

In a preferred embodiment of the invention, the topical pharmaceutical preparation of the invention is a semisolid pharmaceutical preparation, with one of the excipients being polyethylene glycol, in particular polyethylene glycol 400.

Also described herein is a transdermal therapeutic system (TTS), for example a system as described in Pharmazeutische Technologie: Moderne Arzneiformen, Wissenschaftliche Verlagsgesellschaft mbH Stuttgart 1997, pages 81 et sec. TTSs are characterized in principle by a defined supply of medicinal substance to the skin, a total dose of the medicinal substance in the TTS, a total area and an area which is possibly different therefrom for release of the medicinal substance, a covering sheet (backing layer) which is impermeable to the medicinal substance, a medicinal substance reservoir, a control element which controls the supply of medicinal substance to the skin, a (pressure-sensitive) adhesive layer and a detachable protective layer. It is possible on occasions for more than one function to be fulfilled by one and the same element, e.g. reservoir, control and adhesive functions by a suitable adhesive matrix. From the viewpoint of pharmaceutical technology, TTSs are categorized according to the way the control function is achieved, that is to say how it controls the supply of medicinal substance to the skin. Examples which are mentioned here are TTSs with membrane permeation-controlled release (membrane moderated drug delivery), TTSs with matrix diffusion-controlled release and TTSs with microreservoir solution-controlled release.

TTSs with membrane permeation-controlled release are characterized by a polymer membrane composed of a PVA-VA copolymer (Chronomer®) which controls the permeation of the medicinal substance from the reservoir into the skin. The medicinal substance is initially in the form of solid particles or as a dispersion or solution in the reservoir. The polymer membrane can be attached to the reservoir in various ways (extrusion, encapsulation, microencapsulation). TTSs with matrix diffusion-controlled release have a comparatively simpler structure. They contain no separate control element. The release of medicinal substance is controlled by a lipophilic or hydrophilic polymer matrix and/or the adhesive layer. It is possible to distinguish, according to the characteristics of the matrix, between TTSs with a matrix in gel form and TTSs which represent solid polymer laminates. The medicinal substance reservoir is formed by the medicinal substance dissolved in the matrix (monolithic system) or a homogeneous dispersion of solid medicinal substance particles. A matrix TTS can be produced by mixing the medicinal substance particles with a viscous liquid or semisolid polymer at room temperature, followed by crosslinking the polymer chains. A further possibility is also to mix the medicinal substance at elevated temperature with softened polymer (hot melt technique), or the two components (dissolved in an organic solvent) are mixed together and the solvent is then removed in vacuo (solvent evaporation). Shaping is possible by pouring into suitable moulds, spreading with special devices (knives) or by extrusion. In the case of TTSs with microreservoir solution-controlled release (microsealed drug delivery, MDD principle), numerous microcompartments containing the active ingredient and 10-200 µm in size are embedded in a matrix which represents both reservoir and delivery-control element. Because of the matrix, these TTSs are actually assigned to the matrix systems. For production, the medicinal substance is initially dispersed together with water and 40% polyethylene glycol 400 in isopropyl palmitate, which acts as permeation promoter. The resulting dispersion is incorporated by using a special high-energy dispersion technique into a viscous silicone elastomer which simultaneously undergoes catalytic polymerization. The medicinal substance-containing matrix can be shaped specifically by melt or extrusion techniques before it is combined with the carrier in the manner already described. Depending on the physicochemical properties of the medicinal substances and the intended liberation, it is possible to cover the matrix with a layer of a biocompatible polymer in order thus to modify the mechanism and the rate of liberation.

Also described herein is a dosage form for use on the eye (ophthalmologicals). Examples which may be mentioned in this connection are eyebaths or eye lotions, eye inserts, eye ointments, eye sprays, eye drops, preparations for intraocular injection and eyelid ointments. Eye drops may comprise aqueous or oily suspensions of the active ingredient. In this connection, the particle size of the active ingredient employed may be 90% less than 10 µm.

In the case of aqueous suspensions, suspension stabilizers such as, for example, substituted celluloses (e.g. methylcellulose, hydroxypropylmethylcellulose), polyvinyl alcohol, polyvinylpyrrolidone, in addition to preservatives (e.g. chlorocresol, phenylmercury compounds, phenylethanol, benzalkonium chloride or mixtures of individual components) and, where appropriate, sodium chloride to adjust to isotonicity may be used. In the case of oily eye drops, castor oil, peanut oil or medium chain length triglycerides may be employed. It is possible in the case of eye ointments to use ointment bases which have the following properties: sterility or extremely low microbe content, non-irritating, good activity, good distribution of the active ingredient or its solution in the ointment, suppleness, rapid dispersion as fine film over the eyeball, good adhesion to the eye, good stability and low impairment of vision. Hydrocarbon- or cholesterol-containing bases may therefore be employed for eye ointments. In the case of petrolatum, liquid paraffin may be added for consistency reasons. To achieve good spreading, compositions of limited viscosity may be employed. The viscosity at 32°C may be below 1 000 mPa.s, and the yield point may be below 300 mPa. In the case of suspension ointments 90% of the active ingredient particles may be below 10 µm, and no particles above 90 µm should occur. In the case of water/oil emulsion ointments, preservatives such as benzalkonium chloride, thiomersal or phenylethyl alcohol may be added.

### Examples

### Production of the topically applicable pharmaceutical preparations of the invention

### Example 1

550 grams contain

| | | |
|---|---|---|
| Polyethylene glycol 400 | | 440.00 g |
| Carbopol 934® | | 8.25 g |
| Roflumilast | | 1.375 g |
| Sodium hydroxide solution q.s. | | |
| Purified water | to | 550.00 g |

Production takes place by dissolving the active ingredient in the stated amount of polyethylene glycol at about 60-70°C. About 90 grams of purified water are added and mixed homogeneously, and the Carbopol 934 is homogeneously dispersed therein with a high-speed stirrer. While stirring slowly, sodium hydroxide solution is added until a pH of 6.5-7.5 is reached. The remaining water is added up to the final weight and homogeneously mixed.

### Example 2

550 grams contain

| | | |
|---|---|---|
| Roflumilast | | 1.65 g |
| Polyethylene glycol 400 | | 440.00 g |
| Polyethylene glycol 4000 | to | 550.0 g |

Production takes place by treating the two polyethylene glycols to 70°C to give a clear melt. The active ingredient is added likewise to give a clear solution. The preparation is cooled to room temperature while stirring slowly.

### Example 3

550 grams contain

| | | |
|---|---|---|
| Roflumilast | | 1.10 g |
| Tego Care 150® (Th. Goldschmidt) | | 27.50 g |
| Neutral oil (Miglyol 812®) | | 137.50 g |
| Polyethylene glycol 400 | | 275.00 g |
| Cetostearyl alcohol | | 11.00 g |
| Purified water | to | 550 g |

Production takes place by making a clear solution of the neutral oil, the cetostearyl alcohol and Tego Care 150 at about 70°C. The polyethylene glycol, in which the roflumilast has been dissolved, is likewise stirred in using a high-speed stirrer. The water heated to 70°C is added to the lipid phase. A Turrax is used for homogenization. The preparation is then stirred until cold (room temperature).

### Example 4

100 grams contain

| | | |
|---|---|---|
| Roflumilast | | 0.25 g |
| Neutral oil (Miglyol 812®) | | 16.00 g |
| Glycerol monostearate | | 8.00 g |
| Cremophor A6® (BASF) | | 4.00 g |
| Polyethylene glycol 400 | | 62.50 g |
| Purified water | to | 100.00 g |

Production takes place by heating all the components (apart from water) together to about 70-80°C to give a clear solution. The water is then added while stirring, and the preparation produced in this way is cooled to room temperature while stirring.

### Example 5

100 grams contain

| | | |
|---|---|---|
| Roflumilast | | 0.25 g |
| Liquid paraffin | | 15.00 g |
| Wool wax | | 5.00 g |
| White petrolatum | to | 100 g |

Production takes place by making a clear melt of the liquid paraffin, the wool wax and the white petrolatum at about 80°C. The micronized active ingredient is added, and the preparation is stirred until it has cooled to room temperature.

### Example 6

| | | |
|---|---|---|
| Roflumilast | | 0.10 g |
| Liquid paraffin | | 10.00 g |
| Wool wax | | 5.00 g |
| White petrolatum | to | 100 g |

Production takes place in analogy to Example 5.

### Example 7

| | | |
|---|---|---|
| Roflumilast | | 0.10 g |
| Neutral oil (Miglyol 812®) | | 16.00 g |
| Glycerol monostearate | | 8.00 g |
| Cremophor A6® (BASF) | | 2.00 g |
| Polyethylene glycol 400 | | 62.50 g |
| Purified water | to | 100.00 g |

Production takes place in analogy to Example 4.

### Example 8

| | | |
|---|---|---|
| Roflumilast | | 0.10 g |
| Neutral oil ( Miglyol 812®) | | 16.00 g |
| Glycerol monostearate | | 8.00 g |
| Cremophor A6® (BASF) | | 4.00 g |
| Polyethylene glycol 400 | | 62.50 g |
| Purified water | to | 100.00 g |

Production takes place in analogy to Example 4.

### Example 9

Composition of an eye ointment (quantity for 1 000 grams)

| | |
|---|---|
| Roflumilast | 1 g |
| Cetyl alcohol | 4 g |
| High-viscosity paraffin | 200 g |
| White petrolatum | 795 g |

Production: A clear melt of the cetyl alcohol, the high-viscosity paraffin and the white petrolatum is prepared at about 70°C. The micronized roflumilast (90% of the particles below 10 µm) is stirred in, and a homogeneous dispersion is prepared using an Ultra-Turrax. The suspension is cooled to room temperature while stirring and used to fill suitable tubes.

### Example 10 (for reference only)

Composition of a drop solution in the form of an emulsion (quantity for 1 000 millilitres)

| | |
|---|---|
| Roflumilast | 1.5 g |
| Medium chain length triglycerides | 100.0 g |
| Lecithin | 12.0 g |
| Glycerol | 25.0 g |
| Thiomersal | 0.1 g |
| Purified water | to 1 000 ml |

Production: First the roflumilast and then the lecithin are dissolved in the medium chain length triglycerides and the glycerol at 30°C-40°C. While stirring vigorously, the purified water is added and then homogenized until the droplet size of the disperse phase is below 500 nm. The thiomersal is dissolved by stirring. The emulsion is filtered through a 0.45 µm filter and dispensed into suitable containers.

### Example 11 (for reference only)

Composition of a nose ointment (quantity for 1 000 grams)

| | |
|---|---|
| Roflumilast | 1 g |
| Cetyl alcohol | 4 g |
| Wool wax | 50 g |
| High-viscosity paraffin | 200 g |
| White petrolatum | 745 g |

Production: A clear melt of the cetyl alcohol, the high-viscosity paraffin, the wool wax and the white petrolatum is prepared at about 70°C. The micronized roflumilast (90% of the particles below 10 *µ*m) is stirred in, and a homogeneous dispersion is prepared using an Ultra-Turrax. The suspension is cooled to room temperature while stirring and used to fill suitable tubes.

### Investigations of the pharmacokinetics of the topical pharmaceutical preparations

### Comparison of pharmacokinetics parameters of topical pharmaceutical preparations of the invention with oral form

### Example A

A preparation corresponding to Example 7 and a preparation corresponding to Example 8 containing [¹⁴C]roflumilast were applied to shaven areas of rat skin (5 male Wistar rats) 4 cm² in size. The radioactivity concentrations were measured in the plasma after 1 h, 4 h, 8 h, 24 h and in the urine (0-24 h) (n = 5). The dose was 1.7 mg/kg.

### Results:

Preparation of Example 7: Cmax: 0.214 mg equiv./l, AUC(0-24 h): 4.13 (mg equiv./l × h)
Preparation of Example 8: Cmax: 0.214 mg equiv./l, AUC(0-24 h): 3.99 (mg equiv./l × h)

The results standardized to 1 mg/kg are
Preparation of Example 7: Cmax: 0.126, AUC: 2.43
Preparation of Example 8: Cmax: 0.126, AUC: 2.35

Comparison with kinetic parameters after oral administration of 1 mg/kg:
Cmax: 0.225 mg equiv./l, AUC(0-24 h): 3.10 (mg equiv./l × h)

The ratio of the AUC (preparation of Example 7) to the AUC (oral) is 78% and that of the AUC (preparation of Example 8) to the AUC (oral) is 76%.

Results of comparison of the excretions with the urine:
Preparation of Example 7: 19.4% of the dose
Preparation of Example 8: 18.0% of the dose
Oral administration: 18.4% of the dose

### Conclusion:

After percutaneous administration of 1.7 mg/kg [¹⁴C]roflumilast to rats, the total radioactivity is transported well through the skin and reaches a maximum plasma level of 0.214 mg equiv./l after 4 h, irrespective of the preparation employed. Based on the total radioactivity, the AUCs and the excretions with the urine after percutaneous administration are negligibly different from those after oral administration.

### Example B

A preparation corresponding to Example 5 containing [¹⁴C]roflumilast was applied to a shaven area of rat skin (male Wistar rat) 4 cm² in size. The radioactivity concentrations were measured in the plasma after 1 h, 4 h, 8 h, 24 h and in the urine (0-24 h) (n = 5). The dose was 1.77 mg/kg.
Preparation of Example 5: Cmax: 0.331 mg equiv./l, AUC(0-24 h): 4.99 (mg equiv./l × h)

The results standardized to 1 mg/kg are
Preparation of Example 5: Cmax: 0.187, AUC: 2.82

Comparison with kinetic parameters after oral administration of 1 mg/kg:
Cmax: 0.225 mg equiv./l, AUC(0-24 h): 3.10 (mg equiv./l × h)

Results of comparison of the excretions with the urine:
Preparation of Example 5: 22.0% of the dose
Oral administration: 18.4% of the dose

### Conclusion:

These data show that roflumilast is absorbed from the preparation of Example 5 even somewhat better than from the preparations corresponding to Example 7 or 8. The excretion with the urine in the 24 h after administration is 22%, which is also in the region of the excretion with the urine after dermal administration of the preparations corresponding to Example 7 or 8. Comparison with oral administration shows that, irrespective of the composition of the topical preparation, similar Cmax and AUCs and similar excretions with the urine are achieved.

### Industrial applicability

The topically applicable pharmaceutical preparations of the invention can be employed for the systemic treatment and prevention of all diseases regarded as treatable or preventable through the use of PDE 4 inhibitors. Selective cyclic nucleotide phosphodiesterase (PDE) inhibitors (specifically of type 4) are suitable on the one hand as bronchial therapeutic agents (for the treatment of airway obstructions owing to their dilating effect but also owing to their effect increasing the respiratory rate and respiratory drive) and for eliminating erectile dysfunction owing to the vasodilating effect, but on the other hand especially for the treatment of disorders, especially of an inflammatory nature, e.g. of the airways (asthma prophylaxis), of the skin, of the central nervous system, of the intestine, of the eyes and of the joints, which are promoted by mediators such as histamine, PAF (platelet-activating factor), arachidonic acid derivatives such as leukotrienes and prostaglandins, cytokines, interleukins, chemokines, alpha-, beta- and gamma-interferon, tumor necrosis factor (TNF) or oxygen free radicals and proteases.

The invention therefore also relates further to roflumilast, salts of roflumilast, the N-oxide of roflumilast or salts thereof for use in the systemic treatment of diseases regarded as treatable or preventable through use of PDE 4 inhibitors, wherein said treatment comprises administration of roflumilast, salts of roflumilast, the N-oxide of roflumilast and salts thereof in the topically applicable pharmaceutical preparation defined in Claim 1, and wherein said administration is dermal administration. Mention may preferably be made in this connection of psoriasis (vulgaris), toxic and allergic contact eczema, atopic eczema, seborrhoeic eczema, lichen simplex, sunburn, pruritus in the genitoanal region, alopecia areata, hypertrophic scars, discoid lupus erythematosus, follicular and extensive pyodermas, endogenous and exogenous acne, and acne rosacea. Additionally, mention may preferably be made in this connection of bronchitis, allergic bronchitis, bronchial asthma, COPD, rheumatoid arthritis, rheumatoid spondylitis, and osteoarthritis.

The good systemic availability surprisingly observed on topical administration makes the pharmaceutical preparations of the invention suitable for systemic treatment and thus for the treatment of all other diseases which are regarded as treatable or preventable through application of PDE 4 inhibitors, especially the abovementioned diseases.

The topically applicable pharmaceutical preparations of the invention are moreover particularly suitable for administration to groups of patients who are suffering from the abovementioned diseases and have problems in taking pharmaceutical preparations to be administered orally, such as, for example, bed-ridden patients, patients in intensive medical care, patients with swallowing difficulties and children.

The dosage forms of the invention comprise the active pharmaceutical ingredient in the dose customary for the treatment of the particular disease. The dosage of the active ingredient is of the order of magnitude customary for PDE inhibitors, it being possible to administer the daily dose in one or more dosage units. Customary dosages are disclosed for example in WO 95/01338. The normal dose on systemic therapy (oral) is between 0.001 and 3 mg per kilogram and day. Dosage forms preferred according to the invention for topical administration contain from 0.005 mg to 5 mg of roflumilast, preferably from 0.01 mg to 2.5 mg, particularly preferably 0.1 mg to 0.5 mg of roflumilast per dosage unit. Examples of pharmaceutical preparations of the invention contain 0.01 mg, 0.1 mg, 0.125 mg, 0.25 mg and 0.5 mg of roflumilast per dosage unit.

## Claims

1. Topically applicable pharmaceutical preparation consisting of an active pharmaceutical ingredient together with one or more pharmaceutical carriers and/or excipients suitable for topical administration, wherein:
i) the active pharmaceutical ingredient is a compound selected from the group consisting of roflumilast, salts of roflumilast, the N-oxide of the pyridine residue of roflumilast or salts thereof,
ii) the proportion of active pharmaceutical ingredient is up to 1% by weight of the finished pharmaceutical preparation, and
iii) the topically applicable pharmaceutical preparation is a semisolid dosage form selected from the group of ointments (e.g. solution ointment, suspension ointment), creams, gels or pastes.

2. Topically applicable pharmaceutical preparation according to Claim 1, wherein roflmilast is a compound of the formula I 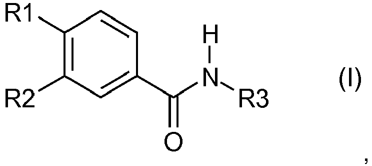 in which
R1 is difluoromethoxy,
R2 is cyclopropylmethoxy and
R3 is 3,5-dichloropyrid-4-yl.

3. Roflumilast, salts of roflumilast, the N-oxide of roflumilast or salts thereof for use in the systemic treatment of diseases regarded as treatable or preventable through use of PDE 4 inhibitors, wherein said treatment comprises administration of roflumilast, salts of roflumilast, the N-oxide of roflumilast and salts thereof in the topically applicable pharmaceutical preparation defined in Claim 1, and wherein said administration is dermal administration.

4. Roflumilast, salts of roflumilast, the N-oxide of roflumilast or salts thereof for use as claimed in claim 3, wherein the diseases regarded as treatable or preventable through use of PDE 4 inhibitors are selected from bronchitis, allergic bronchitis, bronchial asthma, COPD, rheumatoid arthritis, rheumatoid spondylitis and osteoarthritis.

5. Roflumilast, salts of roflumilast, the N-oxide of roflumilast or salts thereof for use as claimed in claim 4, wherein the diseases regarded as treatable or preventable through use of PDE 4 inhibitors is COPD.

6. Roflumilast, salts of roflumilast, the N-oxide of roflumilast or salts thereof for use as claimed in claim 3, wherein the diseases regarded as treatable or preventable through use of PDE 4 inhibitors are selected from psoriasis (vulgaris), toxic and allergic contact eczema, atopic eczema, seborrhoeic eczema, lichen simplex, sunburn, pruritus in the genitoanal region, alopecia areata, hypertrophic scars, discoid lupus erythematosus, follicular and extensive pyodermas, endogenous and exogenous acne, and acne rosacea.

7. Roflumilast, salts of roflumilast, the N-oxide of roflumilast or salts thereof for use as claimed in claim 6, wherein the diseases regarded as treatable or preventable through use of PDE 4 inhibitors is psoriasis.

8. Roflumilast, salts of roflumilast, the N-oxide of roflumilast or salts thereof for use as claimed in claim 6, wherein the diseases regarded as treatable or preventable through use of PDE 4 inhibitors is atopic eczema.

## Patentansprüche

1. Topisch anwendbare pharmazeutische Zubereitung, bestehend aus einem pharmazeutischen Wirkstoff zusammen mit einem oder mehreren für die topische Verabreichung geeigneten pharmazeutischen Trägern und/oder Exzipienten, wobei:
i) es sich bei dem pharmazeutischen Wirkstoff um eine aus der aus Roflumilast, Salzen von Roflumilast, dem N-Oxid des Pyridinrestes von Roflumilast oder Salzen davon bestehenden Gruppe ausgewählte Verbindung handelt,
ii) der Anteil an pharmazeutischem Wirkstoff bis zu 1 Gew.-% der fertigen pharmazeutischen Zubereitung beträgt und
iii) es sich bei der topisch anwendbaren pharmazeutischen Zubereitung um eine aus der Gruppe der Salben (z.B. Lösungssalbe, Suspensionssalbe), Cremen, Gele und Pasten ausgewählte halbfeste Dosierungsform handelt.

2. Topisch anwendbare pharmazeutische Zubereitung nach Anspruch 1, wobei es sich bei Roflumilast und eine Verbindung der Formel I handelt 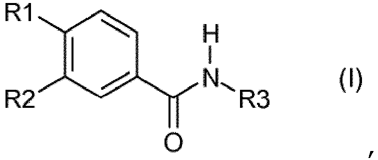 in welcher
R1 für Difluormethoxy steht,
R2 für Cyclopropylmethoxy steht und
R3 für 3,5-Dichlorpyrid-4-yl steht.

3. Roflumilast, Salze von Roflumilast, das N-Oxid von Roflumilast oder Salze davon zur Verwendung bei der systemischen Behandlung von als durch die Anwendung von PDE 4-Inhibitoren als behandelbar oder verhinderbar angesehenen Krankheiten, wobei die Behandlung die Verabreichung von Roflumilast, Salzen von Roflumilast, dem N-Oxid von Roflumilast und Salzen davon in der in Anspruch 1 definierten topisch anwendbaren pharmazeutischen Zubereitung umfasst und wobei es sich bei der Verabreichung um eine dermale Verabreichung handelt.

4. Roflumilast, Salze von Roflumilast, das N-Oxid von Roflumilast oder Salze davon zur Verwendung nach Anspruch 3, wobei die durch die Anwendung von PDE 4-Inhibitoren als behandelbar oder verhinderbar angesehenen Krankheiten aus Bronchitis, allergischer Bronchitis, Bronchialasthma, COPD, rheumatoider Arthritis, rheumatoider Spondylitis und Osterarthritis ausgewählt sind.

5. Roflumilast, Salze von Roflumilast, das N-Oxid von Roflumilast oder Salze davon zur Verwendung nach Anspruch 4, wobei die durch die Anwendung von PDE 4-Inhibitoren als behandelbar oder verhinderbar angesehene Krankheit COPD ist.

6. Roflumilast, Salze von Roflumilast, das N-Oxid von Roflumilast oder Salze davon zur Verwendung nach Anspruch 3, wobei die durch die Anwendung von PDE 4-Inhibitoren als behandelbar oder verhinderbar angesehenen Krankheiten aus Psoriasis (vulgaris), toxischem und allergischem Kontaktekzem, atopischem Ekzem, seborrhoeischem Ekzem, Lichen simplex, Sonnenbrand, Pruritus im genitoanalen Bereich, Alopecia areata, hypertrophen Narben, discoidem Lupus erythematodes, follikulären und extensiven Pyodermien, endogener und exogener Akne und Acne rosacea ausgewählt sind.

7. Roflumilast, Salze von Roflumilast, das N-Oxid von Roflumilast oder Salze davon zur Verwendung nach Anspruch 6, wobei die durch die Anwendung von PDE 4-Inhibitoren als behandelbar oder verhinderbar angesehene Krankheit Psoriasis ist.

8. Roflumilast, Salze von Roflumilast, das N-Oxid von Roflumilast oder Salze davon zur Verwendung nach Anspruch 6, wobei die durch die Anwendung von PDE 4-Inhibitoren als behandelbar oder verhinderbar angesehene Krankheit atopisches Ekzem ist.

## Revendications

1. Préparation pharmaceutique applicable par voie topique constituée d'une substance pharmaceutique active conjointement avec un ou plusieurs véhicules et/ou excipients pharmaceutiques adaptés pour administration topique, dans laquelle :
i) la substance pharmaceutique active est un composé choisi dans le groupe constitué du roflumilast, de sels de roflumilast, du N-oxyde du résidu pyridine du roflumilast ou des sels de celui-ci,
ii) la proportion de substance pharmaceutique active peut aller jusqu'à 1 % en poids de la préparation pharmaceutique finale, et
iii) la préparation pharmaceutique applicable par voie topique est une forme pharmaceutique semi-solide choisie dans le groupe des pommades (par exemple une pommade en solution, une pommade en suspension), des crèmes, des gels ou des pâtes.

2. Préparation pharmaceutique topique selon la revendication 1, dans laquelle le roflumilast est un composé de formule I 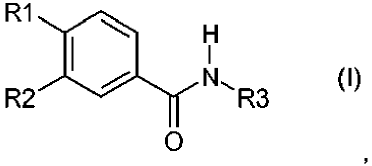 dans laquelle
R1 est difluorométhoxy,
R2 est cyclopropylméthoxy et
R3 est 3,5-dichloropyrid-4-yle.

3. Roflumilast, sels de roflumilast, N-oxyde de roflumilast ou sels de celui-ci utilisés dans le traitement systémique de maladies considérées comme pouvant être traitées ou évitées par utilisation d'inhibiteurs de PDE 4, ledit traitement comprenant l'administration de roflumilast, sels de roflumilast, N-oxyde de roflumilast et sels de celui-ci dans la préparation pharmaceutique applicable par voie topique définie dans la revendication 1, et ladite administration étant une administration par voie cutanée.

4. Roflumilast, sels de roflumilast, N-oxyde de roflumilast ou sels de celui-ci pour utilisation selon la revendication 3, les maladies considérées comme pouvant être traitées ou évitées par utilisation d'inhibiteurs de PDE 4 étant choisies parmi la bronchite, la bronchite allergique, l'asthme bronchique, BPCO, la polyarthrite rhumatoïde, la spondylarthrite rhumatoïde et l'arthrose.

5. Roflumilast, sels de roflumilast, N-oxyde de roflumilast ou sels de celui-ci pour utilisation selon la revendication 4, les maladies considérées comme pouvant être traitées ou évitées par utilisation d'inhibiteurs de PDE 4 étant BPCO.

6. Roflumilast, sels de roflumilast, N-oxyde de roflumilast ou sels de celui-ci selon la revendication 3, les maladies considérées comme pouvant être traitées ou évitées par utilisation d'inhibiteurs de PDE 4 étant choisies parmi le psoriasis (vulgaris), l'eczéma de contact toxique et allergique, l'eczéma atopique, l'eczéma séborrhéique, le lichen simplex, les coups de soleil, le prurit dans la région génito-anale, alopécie aerata, cicatrices hypertrophiques, le lupus érythémateux discoïde, les pyodermites folliculaire et extensive, l'acné endogène et exogène et l'acné rosacée.

7. Roflumilast, sels de roflumilast, N-oxyde de roflumilast ou sels de celui-ci pour utilisation selon la revendication 6, les maladies considérées comme pouvant être traitées ou évitées par utilisation d'inhibiteurs de PDE 4 étant le psoriasis.

8. Roflumilast, sels de roflumilast, N-oxyde de roflumilast ou sels de celui-ci pour l'utilisation selon la revendication 6, les maladies considérées comme pouvant être traitées ou évitées par utilisation d'inhibiteurs de PDE 4 étant l'eczéma atopique.
